(19) Europäisches Patentamt
European Patent Office
Office européen des brevets

(11) **EP 2 380 498 B1**

(12) **EUROPEAN PATENT SPECIFICATION**

(45) Date of publication and mention of the grant of the patent:
**03.12.2014 Bulletin 2014/49**

(51) Int Cl.:
*A61B 8/06* (2006.01)   *G01S 15/89* (2006.01)

(21) Application number: **11163392.1**

(22) Date of filing: **21.04.2011**

(54) **Adaptive clutter filtering in an ultrasound system**

Adaptive Clutterfilterung in einem Ultraschallsystem

Filtrage d'écho adaptatif dans un système à ultrasons

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **26.04.2010 KR 20100038422**
**03.03.2011 KR 20110018707**

(43) Date of publication of application:
**26.10.2011 Bulletin 2011/43**

(73) Proprietor: **Samsung Medison Co., Ltd.**
**Nam-myun**
**Hongchun-gun**
**Kangwon do 250-875 (KR)**

(72) Inventors:
• **Bae, Moo Ho**
**138-240, Seoul (KR)**
• **Lee, Young Seok**
**200-760, Gangwon-do (KR)**
• **Park, Sung Bae**
**200-060, Gangwon-Do (KR)**
• **Lee, Kyoung Bo**
**465-816, Gyeonggi-do (KR)**

(74) Representative: **Schmid, Wolfgang**
**Lorenz & Kollegen**
**Patentanwälte Partnerschaftsgesellschaft mbB**
**Alte Ulmer Strasse 2**
**89522 Heidenheim (DE)**

(56) References cited:
• **YOUNG BOK AHN ET AL: "ESTIMATION OF MEAN FREQUENCY AND VARIANCE OF ULTRASONIC DOPPLER SIGNAL BY USING SECOND-ORDER AUTOREGRESSIVE MODEL", IEEE TRANSACTIONS ON ULTRASONICS, FERROELECTRICS AND FREQUENCY CONTROL, IEEE, US, vol. 38, no. 3, 1 May 1991 (1991-05-01), pages 172-182, XP000206313, ISSN: 0885-3010, DOI: 10.1109/58.79600**
• **XIAOTAO WANG ET AL: "An Novel Clutter Rejection Filters Applied to Wideband Blood Flow Velocity Estimation", ENGINEERING IN MEDICINE AND BIOLOGY SOCIETY, 2005. IEEE-EMBS 2005. 27T H ANNUAL INTERNATIONAL CONFERENCE OF THE SHANGHAI, CHINA 01-04 SEPT. 2005, PISCATAWAY, NJ, USA,IEEE, 1 January 2005 (2005-01-01), pages 7289-7292, XP031000887, DOI: 10.1109/IEMBS.2005.1616194 ISBN: 978-0-7803-8741-6**
• **PEIDONG WANG ET AL: "An Improved Mean Frequency Estimator for Ultrasonic Color Flow Imaging Using Second-Order Autoregressive Model", ENGINEERING IN MEDICINE AND BIOLOGY SOCIETY, 2005. IEEE-EMBS 2005. 27T H ANNUAL INTERNATIONAL CONFERENCE OF THE SHANGHAI, CHINA 01-04 SEPT. 2005, PISCATAWAY, NJ, USA,IEEE, 1 September 2005 (2005-09-01), pages 5643-5646, XP010907173, DOI: 10.1109/IEMBS.2005.1615766 ISBN: 978-0-7803-8741-6**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

TECHNICAL FIELD

The present disclosure generally relates to strain imaging, and more particularly to adaptive clutter filtering in an ultrasound system.

BACKGROUND

An ultrasound system has become an important and popular diagnostic tool since it has a wide range of applications. Specifically, due to its non-invasive and non-destructive nature, the ultrasound system has been extensively used in the medical profession. Modem high-performance ultrasound systems and techniques are commonly used to produce two or three-dimensional images of internal features of an object (e.g., human organs).

Generally, the ultrasound system may operate in a Brightness-mode (B-mode) visualizing reflectivity of an ultrasound signal reflected from a target object as a 2-dimensional image, a Doppler mode visualizing a velocity of a moving object as spectral Doppler by using the Doppler effect, a color Doppler mode visualizing velocity and direction of a moving object with colors by using the Doppler effect and an elasticity mode visualizing mechanical characteristics of tissues such as the elasticity of the same.

In the color Doppler mode, the ultrasound system may transmit an ultrasound signal to the target object and receive the ultrasound echoes to thereby form a Doppler signal. The ultrasound system may form a color Doppler image based on the Doppler signal. The Doppler signal may include a low frequency signal (the so-called clutter signal) due to the motion of a cardiac wall or valve of a heart and a noise in addition to a signal caused by a blood flow (referred to as "blood flow signal"). The clutter signal may have amplitude, which is over 100 times than that of the blood flow signal. The clutter signal may be an obstacle to accurately detect a velocity of the blood flow. Thus, it is required to remove the clutter signal from the Doppler signal to accurately detect the velocities of the blood flow.

A frequency down mixing method has been used to remove the clutter signal. According to the frequency down mixing method, frequency components corresponding to the clutter signal are estimated and then down mixing, i.e., frequency shifting is performed upon the Doppler signal such that a center frequency of the clutter signal becomes zero. Thereafter, the clutter filtering is performed to remove the clutter signal.

Generally, the ultrasound system may acquire an amount of the ultrasound data by the ensemble number and estimate the frequency components corresponding to the clutter signal by using the acquired ultrasound data. However, it may be difficult to accurately estimate the frequency components corresponding to the clutter signal and frequency components corresponding to the blood flow signal by using the ultrasound data corresponding to the limited ensemble number.

To cope with the above problem, the conventional clutter filtering has been performed by setting a high cutoff frequency of a high pass filter. When the cutoff frequency is set high, a Doppler signal (i.e., blood flow signal) corresponding to a blood flow of a relatively low speed may be removed and a clutter signal of a relatively high frequency may not be removed. Thus, the motion of the blood flow may not be accurately indicated on a color Doppler image.

XP000206313 "Estimation of Mean Frequency and Variance of Ultrasonic Doppler Signal by Using Second-Order Autoregressive Model" by Y. B. Ahn et al., forming the closest prior art document for the invention, refers to an ultrasound system and a method for performing cluttering filtering in this system which proposes a second-order autoregressive model in order to estimate the mean frequency and variance of the diagnostic ultrasound Doppler signal in the presence of clutter noise.

XP031000887 "A Novel Clutter Rejection Filters Applied to Wideband Blood Flow Velocity Estimation" by X. Wang et al. Wang discloses a novel scheme using parameter estimation methods based on the two-dimensional correlation function model and the conventional down mixing to clutter rejection.

XP010907173 "An Improved Mean Frequency Estimator for Ultrasonic Color Flow Imaging Using Second-Order Autoregressive Model" by P. Wang et al and Xiaotao Wang refers to a second-order AR estimator which is capable to first estimate the mean frequency of the clutter signal and then compensate the frequency down-shift of the Doppler signal using this mean frequency.

SUMMARY

Embodiments for adaptively performing clutter filtering in an ultrasound system are disclosed herein. In one embodiment an ultrasound system comprises the features stated in claim 1.

In another embodiment, a method of performing cluttering filtering in an ultrasound system, comprises the features stated in claim 3.

BRIEF DESCRIPTION OF THE DRAWINGS

FIG. 1 is a block diagram showing an illustrative embodiment of an ultrasound system.
FIG. 2 is a schematic diagram showing an example of displaying a brightness mode image and a region of interest.
FIG. 3 is a block diagram showing an illustrative embodiment of an ultrasound data acquisition unit.
FIG. 4 is a flowchart showing an illustrative embodiment of forming a color Doppler image.
FIG. 5 is a flowchart showing an illustrative embodiment of detecting the frequency components corresponding to the clutter signal.

DETAILED DESCRIPTION

A detailed description may be provided with reference to the accompanying drawings.

One of ordinary skill in the art may realize that the following description is illustrative only and is not in any way limiting. Other embodiments of the present invention may readily suggest themselves to such skilled persons having the benefit of this disclosure.

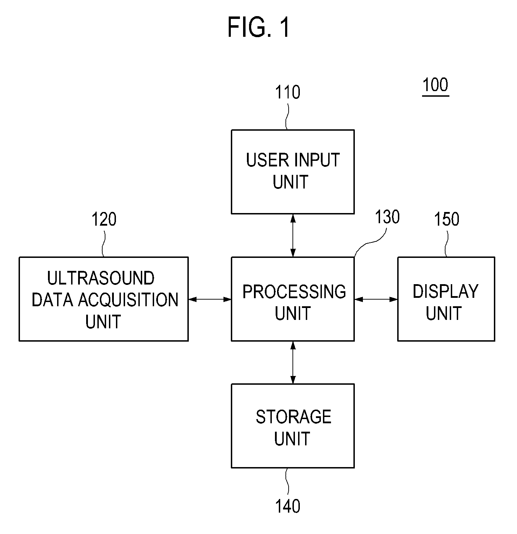

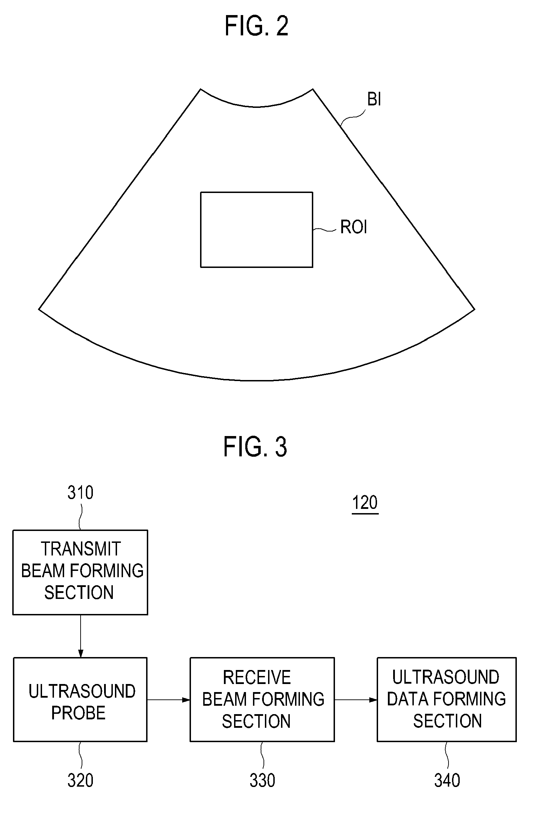

Referring to FIG. 1, an ultrasound system constructed in accordance with one embodiment is shown. The ultrasound system 100 includes a user input unit 110, an ultrasound data acquisition unit 120, a processing unit 130, a storage unit 140 and a display unit 150. The user input unit 110 receives input information of a user. The input information includes input information for setting a region of interest (ROI) on a brightness (B)-mode image BI, as illustrated in FIG. 2. The ROI includes a color box for acquiring ultrasound data for color Doppler imaging. However, the ROI may not be limited thereto. The user input unit 100 includes a control panel, a mouse, a keyboard, a track ball and the like.

The ultrasound data acquisition unit 120 is configured to transmit ultrasound beams to a target object and receive ultrasound echoes reflected from the target object to thereby form ultrasound data representative of the target object. An operation of the ultrasound acquisition unit will be described in detail by referring to FIG. 3.

FIG. 3 is a block diagram showing an illustrative embodiment of the ultrasound data acquisition unit 120. Referring to FIG. 3, the ultrasound data acquisition unit 120 includes a transmit (Tx) signal forming section 310. The Tx signal forming section 310 is configured to generate a plurality of Tx signals. The Tx signal forming section 310 forms a Tx pattern of the Tx signals according to image modes such as a brightness mode, a Doppler mode, a color Doppler mode and the like. In one embodiment, the Tx pattern of the Tx signals include a first Tx pattern for the brightness mode and a second Tx pattern based on an ensemble number for the color Doppler mode. The ensemble number represents the number for transmission/reception of ultrasound signals along one single scan line to acquire Doppler signals.

The ultrasound data acquisition unit 120 further includes an ultrasound probe 320, which is coupled to the Tx signal forming section 210. The ultrasound probe 320 includes an array transducer containing a plurality of transducer elements for reciprocal conversion between electric signals and ultrasound signals. The ultrasound probe 320 is configured to transmit ultrasound signals in response to the Tx signals. In one embodiment, the transmitted ultrasound signals include first ultrasound signals based on the first Tx pattern of the Tx signals and second ultrasound signals based on the second Tx pattern of the Tx signals. The ultrasound probe 320 is be further configured to receive ultrasound echoes reflected from the target object to thereby output receive signals. In one embodiment, the receive signals include first receive signals associated with the first ultrasound signals and second receive signals associated with the second ultrasound signals. The ultrasound probe 320 includes a convex probe, a linear probe and the like.

The ultrasound data acquisition unit 120 further includes a beam forming section 330, which is coupled to the ultrasound probe 320. The beam forming section 330 is configured to digitize the receive signals into digital signals. The beam forming section 330 is configured to apply delays to the digital signals in consideration of distances between the elements of the ultrasound probe 320 and focal points. The beam forming section 330 further sums the delayed digital signals to form receive-focused signals. In one embodiment, the beam forming section 330 forms first receive-focused signals based on the first receive signals and second receive-focused signals based on the second receive signals.

The ultrasound data acquisition unit 120 further includes an ultrasound data forming section 340, which is coupled to the beam forming section 330. The ultrasound data forming section 340 is configured to form ultrasound data based on the receive-focused signals. In one embedment, the ultrasound data forming section 340 forms first ultrasound data for a B-mode image BI. The first ultrasound data are radio frequency data, although it may not be limited thereto. The ultrasound data forming section 340 forms second ultrasound data corresponding to the ROI for a Color Doppler image (i.e., ensemble data). The second ultrasound data include in-phase/quadrature data, although the second ultrasound data may not be limited thereto.

Referring to FIG. 1, the processing unit 130, which is coupled to the ultrasound data acquisition unit 120, may be embodied with at least one of a central processing unit, a microprocessor, a graphic processing unit and the like. However, the processing unit 130 may not be limited thereto.

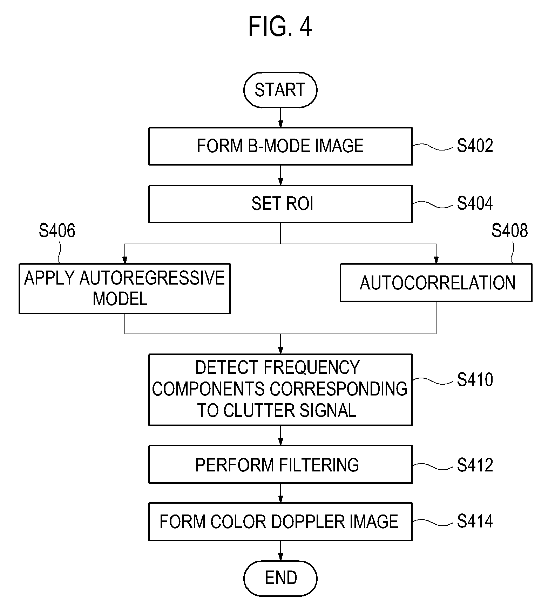

FIG. 4 is a flowchart showing an illustrative embodiment of forming a color Doppler image. Referring to FIG. 4, the processing unit 130 forms a B-mode image BI based on the first ultrasound data, which are provided from the ultrasound data acquisition unit 120 at S402. The B-mode image BI is displayed on the display unit 150 for allowing a user to set the ROI thereon by using the user input unit 110.

If the input information is provided through the user input unit 110, then the processing unit 130 sets the ROI on the B-mode image based on the input information at S404. In response to setting the ROI, the processing unit 130 controls the ultrasound data acquisition unit 120 for operation in the color Doppler mode to thereby obtain the second ultrasound data from the ROI (i.e., ensemble data).

The processing unit 130 is configured to estimate two frequency components and first strengths (or powers) of the second ultrasound data by using the autoregressive (AR) model at S406. Generally, a function H(z) of an $m^{th}$ order AR model may be expressed as the following equation.

$$H(z) = \frac{\sqrt{e}}{1 + a_2 z^{-1} + \cdots + a_{(p+1)} z^{-m}} \qquad (1)$$

wherein a numerator may be represented by a minimized dispersion $e$ and poles, which are roots of a denominator, may be represented by a polynomial of linear prediction coefficients $a_k$ and $z$ .

Linear prediction may be adopted to estimate the linear prediction coefficients $a_k$ of equation (1). The linear prediction is a technique that estimates a current value based on linear sum of previous values of a given signal. Assuming that N discrete signals $(x_n)_{n \in [0,N]}$ are provided, forward linear prediction $y_n$ and backward linear prediction $z_n$ are indicated as linear prediction coefficients $(a_n)_{n \in [1,k]}$ ofk coefficients.

$$y_n = -\sum_{i=1}^{n} a_i x_{n-i}$$
$$z_n = -\sum_{i=1}^{k} a_i x_{n+i} \qquad (2)$$

The forward linear prediction $y_n$ is represented by the minimized *sum $F_k$* of squared errors, as follows.

$$F_k = \sum_{n=k}^{N} (x_n - y_n)^2 = \sum_{n=k}^{N} \left( x_n - (-\sum_{i=1}^{k} a_i x_{n-i}) \right)^2 \qquad (3)$$

Typically, the linear prediction coefficients $(a_n)_{n \in [1,N]}$ are selected through minimization of the sum of squared errors. The backward linear prediction $z_n$ is represented by the minimized sum $B_k$ of squared errors, as follows.

$$B_k = \sum_{n=k}^{N} (x_n - z_n)^2 = \sum_{n=k}^{N} \left( x_n - (-\sum_{i=1}^{k} a_i x_{n+i}) \right)^2 \qquad (4)$$

To estimate the linear prediction coefficients $(a_n)_{n \in [1,N]}$ for minimizing the error of the forward linear prediction or the backward linear prediction, initial state parameters may be stabilized by the Burg's recursion based on the Levinson-Durbin recursion.

If the linear prediction coefficients of the AR model, in which the initial state parameters are stabilized by the Burg's recursion based on the Levinson-Durbin recursion, are estimated, then all poles of the denominator in equation (1) may be estimated.

The processing unit 130 is configured to detect two poles for the second ultrasound data by using the second order AR model and detect frequency components and strengths corresponding to the respective two poles. The second order AR model may be defined as follows.

$$H(z) = \frac{z^2 \sqrt{e}}{z^2 + a_1 z + a_2} = \frac{z^2 \sqrt{e}}{(z - p_1)(z - p_2)} \qquad (5)$$

wherein $p_1$ represents a first pole of the second order AR model function H(z) and $p_2$ represents a second pole of the second order AR model function $H(z)$.

The processing unit 130 is further configured to detect frequency components $\omega_1$ and $\omega_2$ corresponding to the respective two poles of the second ultrasound data as the following equation.

$$\omega_1 = \tan^{-1}\left(\frac{\mathrm{Im}(p_1)}{\mathrm{Re}(p_1)}\right)$$
$$\omega_2 = \tan^{-1}\left(\frac{\mathrm{Im}(p_2)}{\mathrm{Re}(p_2)}\right) \qquad (6)$$

Further, the processing unit 130 is further configured to compute a mean frequency component $\omega_3$ and a second strength (or power) corresponding to the second ultrasound data by using auto-correlation at S408. In another embodiment, the processing unit 130 is configured to compute a mean frequency component $\omega_3$ and a second strength (or power) corresponding to the second ultrasound data by using the fast Fourier transform.

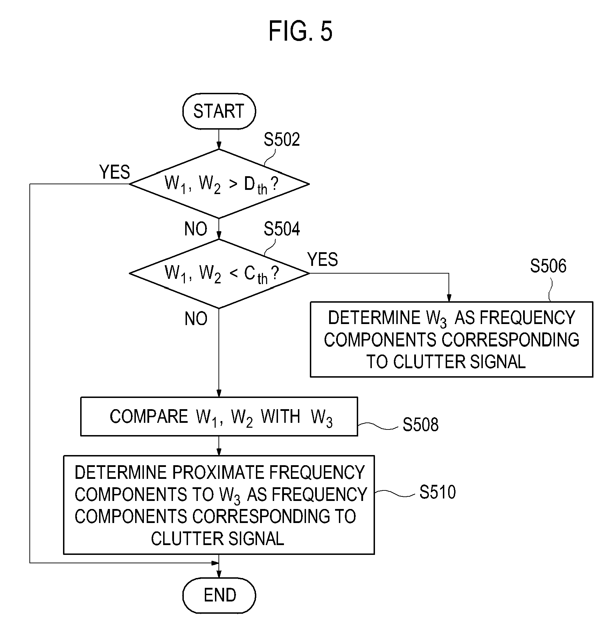

The processing unit 130 is configured to detect frequency components corresponding to the clutter signal by using the frequency components $\omega_1$ and $\omega_2$ and the mean frequency component $\omega_3$ at S410. The step of S410 will be described in detail by referring to FIG. 5.

FIG. 5 is a flowchart showing an illustrative embodiment of detecting the frequency components corresponding to the clutter signal. Referring to FIG. 5, the processing unit 130 is configured to compare the frequency components $\omega_1$ and $\omega_2$, which have been detected by using the second order AR model, with a predetermined Doppler threshold $D_{th}$ at S502. In such a case, the Doppler threshold $D_{th}$ is an arbitrary value estimated by using a plurality of clinical data and set to different values according to parts of organs in the target object.

If it is determined that the frequency components $\omega_1$ and $\omega_2$ are greater than the Doppler threshold $D_{th}$ at S502, then the processing unit 130 is configured to determine that the clutter signal does not exist in the Doppler signal. This is so that the detection of the frequency components corresponding to the clutter signal may not be carried out.

On the other hand, if it is determined that at least one of the frequency components $\omega_1$ and $\omega_2$ is less than the Doppler threshold $D_{th}$ at S502, then the processing unit 130 is configured to compare the frequency components $\omega_1$ and $\omega_2$ with a predetermined clutter threshold $C_{th}$ at S504. If it is determined that the frequency components $\omega_1$ and $\omega_2$ are less than the clutter threshold $C_{th}$ at S502, then the frequency components $\omega_1$ and $\omega_2$ may be considered as a noise and a clutter signal. This is so that the processing unit 130 is configured to detect the mean frequency component $\omega_3$ as the frequency component corresponding to the clutter signal at S506.

However, if it is determined that at least one of the frequency components $\omega_1$ and $\omega_2$ is greater than the Clutter threshold $C_{th}$ at S504, then the processing unit 130 is configured to compare the frequency components $\omega_1$ and $\omega_2$ with the mean frequency component $\omega_3$ at S508. The processing unit 130 is further configured to detect proximate frequency components from the mean frequency component $\omega_3$ as the frequency components corresponding to the clutter signal at S510.

Referring back to FIG. 4, the processing unit 130 is configured to perform filtering upon the second ultrasound data by using the frequency components corresponding to the clutter signal at S412. In one embodiment, the processing unit 130 may be configured to set the frequency components corresponding to the clutter signal as the frequency down mixing frequencies. The processing unit 130 may be further configured to perform the frequency down mixing upon the second ultrasound data based on the down mixing frequencies. The processing unit 130 may be configured to restore the clutter-filtered second ultrasound data to the original frequencies.

Further, if the frequency components corresponding to the clutter signal are not detected, then the processing unit 130 is configured to compare the first strengths and the second strengths for the second ultrasound data to perform noise removal upon the second ultrasound data. The noise removal may be performed by using a well-known method, so that the detailed description thereof will be omitted herein. The processing unit 130 may be configured to form a color Doppler image by using the second ultrasound data with the clutter signal filtered at S414.

Referring to FIG. 1, the storage unit 140, which is coupled to the ultrasound data acquisition unit 120 via the processing unit 130, is configured to store the ultrasound data (first ultrasound data and second ultrasound data) acquired in the ultrasound data acquisition unit 120. Also, the storage unit 140 is configured to store the Doppler threshold $D_{th}$ and the clutter threshold $C_{th}$. Further, the storage unit 140 may further include the second ultrasound data with the clutter signal filtered.

The display unit 150 displays the B-mod image and the color Doppler image, which have been formed in the processing unit 130. The display unit 150 includes at least one of a cathode ray tube (CRT) display, a liquid crystal display (LCD), an organic light emitting diode (OLED) display and the like.

Although embodiments have been described with reference to a number of illustrative embodiments thereof, it should be understood that numerous other modifications and embodiments can be devised by those skilled in the art that will fall within the scope of the principles of this disclosure. More particularly, numerous variations and modifications are possible in the component parts and/or arrangements of the subject combination arrangement within the scope of the disclosure, the drawings and the appended claims. In addition to variations and modifications in the component parts and/or arrangements, alternative uses will also be apparent to those skilled in the art.

## Claims

1. An ultrasound system (100), comprising:

   an ultrasound data acquisition unit (120) configured to perform a transmit/receive operation including transmitting ultrasound signals to a target object and receiving ultrasound echoes reflected from the target object to thereby acquire ultrasound data for color Doppler imaging, and
   a processing unit (130) configured to extract (S406) two frequency components ($\omega_1$, $\omega_2$) from the ultrasound data using a second order autoregressive model, wherein the two frequency components ($\omega_1$, $\omega_2$) correspond to the two poles of the model function of the autoregressive model,
   **characterized in that** the processing unit (130) is further configured to:

   compute (S408) a mean frequency component ($\omega_3$) of the ultrasound data using autocorrelation or fast Fourier transform,
   compare (S502) each of the two frequency components ($\omega_1$, $\omega_2$) with a predetermined Doppler threshold ($D_{th}$),
   determine, when the two frequency components ($\omega_1$, $\omega_2$) are greater than the predetermined Doppler threshold ($D_{th}$), that a clutter signal does not exist in the ultrasound data,
   compare (S504), when at least one of the two frequency components ($\omega_1$, $\omega_2$) is less than the predetermined Doppler threshold ($D_{th}$), each of the two frequency components ($\omega_1$, $\omega_2$) with a predetermined clutter threshold ($C_{th}$),
   compare (S508), when at least one of the two frequency components ($\omega_1$, $\omega_2$) is greater than the predetermined clutter threshold ($C_{th}$), each of the two frequency components ($\omega_1$, $\omega_2$) with the mean frequency component ($\omega_3$) to detect (S510) frequency components proximate to the mean frequency component ($\omega_3$) as the frequency components corresponding to the clutter signal,
   set (S506), when the two frequency components ($\omega_1$, $\omega_2$) are less than the predetermined clutter threshold ($C_{th}$), the mean frequency component ($\omega_3$) as the frequency component corresponding to the clutter signal and
   perform clutter filtering upon the ultrasound data by using the frequency components corresponding to the clutter signal.

2. The ultrasound system (100) of Claim 1, wherein the processing unit (130) is configured to:

   set the frequency components corresponding to the clutter signal as frequency down mixing frequencies,
   perform frequency down mixing upon the ultrasound data based on the frequency down mixing frequencies, and
   perform the clutter filtering upon the frequency down mixed ultrasound data.

3. A method of performing cluttering filtering in an ultrasound system (100), comprising:

   performing a transmit/receive operation including transmitting ultrasound signals to a target object and receiving ultrasound echoes reflected from the target object to thereby acquire ultrasound data for color Doppler imaging, and

extracting (S406) two frequency components ($\omega_1$, $\omega_2$) from the ultrasound data using a second order autoregressive model, wherein the two frequency components ($\omega_1$, $\omega_2$) correspond to the two poles of the model function of the autoregressive model, **characterized by** further comprising:

computing (S408) a mean frequency component ($\omega_3$) of the ultrasound data using autocorrelation or fast Fourier transform;

comparing (S502) each of the two frequency components ($\omega_1$, $\omega_2$) with a predetermined Doppler threshold ($D_{th}$) ;

determining, when the two frequency components ($\omega_1$, $\omega_2$) are greater than the predetermined Doppler threshold ($D_{th}$), that a clutter signal does not exist in the ultrasound data;

comparing (S504), when at least one of the two frequency components ($\omega_1$, $\omega_2$) is less than the predetermined Doppler threshold ($D_{th}$), each of the two frequency components ($\omega_1$, $\omega_2$) with a predetermined clutter threshold ($C_{th}$);

comparing (S508), when at least one of the two frequency components ($\omega_1$, $\omega2$) is greater than the predetennined clutter threshold ($C_{th}$), each of the two frequency components ($\omega_1$, $\omega_2$) with the mean frequency component ($\omega_3$) to detect (S510) frequency components proximate to the mean frequency component ($\omega_3$) as the frequency components corresponding to the clutter signal;

setting (S506), when the two frequency components ($\omega_1$, $\omega_2$) are less than the predetermined clutter threshold ($C_{th}$), the mean frequency component ($\omega_3$) as the frequency component corresponding to the clutter signal; and

performing clutter filtering upon the ultrasound data by using the frequency components corresponding to the clutter signal.

4. The method of Claim 3, further comprising:

setting the frequency components corresponding to the clutter signal as frequency down mixing frequencies, performing frequency down mixing upon the ultrasound data based on the frequency down mixing frequencies, and

performing the clutter filtering upon the frequency down mixed ultrasound data.

**Patentansprüche**

1. Ultraschallsystem (100), welches Folgendes aufweist:

eine Ultraschalldaten-Erlangungseinheit (102), die dafür vorgesehen ist, eine Übertragungs-/Empfangs-Operation einschließlich des Übertragens von Ultraschallsignalen zu einem Zielobjekt und Empfangen von dem Zielobjekt reflektierten Ultraschallechos durchzuführen, um dadurch Ultraschalldaten für ein Farb-Doppler-Abbilden zu erlangen, und

eine Verarbeitungseinheit (130), die dafür vorgesehen ist, zwei Frequenzkomponenten ($\omega_1$, $\omega_2$) von den Ultraschalldaten unter Verwendung eines autoregressiven Modells zweiter Ordnung zu extrahieren (S406), wobei die zwei Frequenzkomponenten ($\omega_1$, $\omega_2$) den zwei Polen der Modellfunktion des autoregressiven Modells entsprechen,

**dadurch gekennzeichnet, dass**

die Verarbeitungseinheit (130) des Weiteren für Folgendes vorgesehen ist:

Berechnen (S408) einer mittleren Frequenzkomponente ($\omega_3$) der Ultraschalldaten unter Verwendung von Autokorrelation oder einer schnellen Fourier-Transformation,

Vergleichen (S502) jeder der zwei Frequenzkomponenten ($\omega_1$, $\omega_2$) mit einem vorbestimmten Doppler-Grenzwert ($D_{th}$),

Festlegen, wenn die zwei Frequenzkomponenten ($\omega_1$, $\omega_2$) größer sind als der vorbestimmte Doppler-Grenzwert ($D_{th}$), dass ein Stör- bzw. Cluttersignal in den Ultraschalldaten nicht existiert,

Vergleichen (S504), wenn wenigstens eine der zwei Frequenzkomponenten ($\omega_1$, $\omega_2$) kleiner ist als der vorbestimmte Doppler-Grenzwert ($D_{th}$), jeder der zwei Frequenzkomponenten ($\omega_1$, $\omega_2$) mit einem vorbestimmten Cluttergrenzwert (Cth),

Vergleichen (S508), wenn wenigstens eine der zwei Frequenzkomponenten ($\omega_1$, $\omega_2$) größer ist als der

vorbestimmte Cluttergrenzwert ($C_{th}$), jeder der zwei Frequenzkomponenten ($\omega_1$, $\omega_2$) mit der mittleren Frequenzkomponente ($\omega_3$),

um Frequenzkomponenten benachbart zu der mittleren Frequenzkomponente ($\omega_3$) als die Frequenzkomponenten zu detektieren, die dem Stör- bzw. Clutter-signal entsprechen,

Festlegen (S506), wenn die zwei Frequenzkomponenten ($\omega_1$, $\omega_2$) niedriger sind als der vorbestimmte Cluttergrenzwert ($C_{th}$), der mittleren Frequenzkomponente ($\omega_3$) als die Frequenzkomponente, welche dem Stör- bzw. Cluttersignal entspricht, und

Durchführen eines Stör- bzw. Clutterfilterns an den Ultraschalldaten unter Verwendung der Frequenzkomponenten, welche dem Stör- bzw. Cluttersignal entsprechen.

2. Ultraschallsystem (100) nach Anspruch 1, wobei die Verarbeitungseinheit (130) für Folgendes vorgesehen ist:

Festlegen der Frequenzkomponenten, welche dem Stör- bzw. Cluttersignal entsprechen, als Frequenz-Abwärtsmisch- bzw. Downmixing-Frequenzen,

Durchführen des Frequenz-Downmixings an den Ultraschalldaten basierend auf den Frequenz-Downmixing-Frequenzen, und

Durchführen des Stör- bzw. Clutterfilterns an den downgemixten Frequenz-Ultraschalldaten.

3. Verfahren zum Durchführen eines Stör- bzw. Clutterfilterns in einem Ultraschallsystem (100), welches Folgendes aufweist:

Durchführen einer Übertragungs-/Empfangs-Operation einschließlich des Übertragens von Ultraschallsignalen zu einem Zielobjekt und Empfangen von von dem Zielobjekt reflektiert an Ultraschallechos, um dadurch Ultraschalldaten für ein Farb-Doppler-Abbilden zu erlangen, und

Extrahieren (S406) zweier Frequenzkomponenten ($\omega_1$, $\omega_2$) von den Ultraschalldaten unter Verwendung eines autoregressiven Modells zweiter Ordnung, wobei die zwei Frequenzkomponenten ($\omega_1$, $\omega_2$) den zwei Polen der Modellfunktion des autoregressiven Modells entsprechen, **gekennzeichnet durch**
das Aufweisen folgender weiterer Schritte:

Berechnen (S408) einer mittleren Frequenzkomponente ($\omega_3$) der Ultraschalldaten unter Verwendung von Autokorrelation oder einer schnellen Fourier-Transformation;

Vergleichen (S502) jeder der zwei Frequenzkomponenten ($\omega_1$, $\omega_2$) mit einem vorbestimmten Doppler-Grenzwert ($D_{th}$);

Festlegen, wenn die zwei Frequenzkomponenten ($\omega_1$, $\omega_2$) größer sind als der vorbestimmte Doppler-Grenzwert ($D_{th}$), dass ein Stör- bzw. Cluttersignal in den Ultraschalldaten nicht existiert,

Vergleichen (S504), wenn wenigstens eine der zwei Frequenzkomponenten ($\omega_1$, $\omega_2$) kleiner ist als der vorbestimmte Doppler-Grenzwert ($D_{th}$), jeder der zwei Frequenzkomponenten ($\omega_1$, $\omega_2$) mit einem vorbestimmten Cluttergrenzwert (Cth),

Vergleichen (S508), wenn wenigstens eine der zwei Frequenzkomponenten ($\omega_1$, $\omega_2$) größer ist als der vorbestimmte Cluttergrenzwert ($C_{th}$), jeder der zwei Frequenzkomponenten ($\omega_1$, $\omega_2$) mit der mittleren Frequenzkomponente ($\omega_3$),

um Frequenzkomponenten benachbart zu der mittleren Frequenzkomponente ($\omega_3$) als die Frequenzkomponenten zu detektieren, die dem Stör- bzw. Cluttersignal entsprechen,

Festlegen (S506), wenn die zwei Frequenzkomponenten ($\omega_1$, $\omega_2$) niedriger sind als der vorbestimmte Cluttergrenzwert ($C_{th}$), die mittlere Frequenzkomponente ($\omega_3$) als die Frequenzkomponente, welche dem Stör- bzw. Cluttersignal entspricht, und

Durchführen eines Stör- bzw. Clutterfilterns an den Ultraschalldaten unter Verwendung der Frequenzkomponenten, welche dem Stör- bzw. Cluttersignal entsprechen.

4. Verfahren nach Anspruch 3, welches des Weiteren Folgendes aufweist:

Festlegen der Frequenzkomponenten, welche dem Stör- bzw. Cluttersignal entsprechen, als Frequenz-Abwärtsmisch- bzw. Downmixing-Frequenzen, Durchführen des Frequenz-Downmixings an den Ultraschalldaten basierend auf den Frequenz-Downmixing-Frequenzen, und

Durchführen des Stör- bzw. Clutterfilterns an den downgemixten Ultraschalldaten.

**Revendications**

1. Système ultrasonique (100) comportant:

   une unité d'acquisition de données ultrasoniques (120) configurée pour effectuer une opération de transmission/réception comprenant la transmission de signaux ultrasoniques sur un objet cible et pour la réception des échos ultrasoniques réfléchis par l'objet cible afin d'acquérir des données ultrasoniques pour obtenir une image Doppler en couleur, et

   une unité de traitement (130) configurée pour extraire (S406) deux composantes de fréquences ($\omega_1$, $\omega_2$) provenant des données ultrasoniques utilisant un modèle autoregressif de second ordre, dans lequel les deux composantes de fréquences ($\omega_1$, $\omega_2$) correspondent aux deux pôles de la fonction modèle du modèle autoregressif,

   **caractérisé en ce que** l'unité de traitement (130) est en outre configurée pour :

   calculer (S408) une composante de fréquence moyenne ($\omega_3$) des données ultrasoniques utilisant l'autocorrélation ou la transformation de Fourrier rapide,

   comparer (S502) chacune des deux composantes de fréquences ($\omega_1$, $\omega_2$) avec un seuil Doppler prédéterminé ($D_{th}$),

   déterminer quand les deux composantes de fréquences ($\omega_1$, $\omega_2$) sont supérieures au seuil Doppler prédéterminé ($D_{th}$), et qu'un signal parasite n'existe pas dans les données ultrasoniques,

   comparer (S504) quand au moins une des deux composantes de fréquences ($\omega_1$, $\omega_2$) est inférieure au seuil Doppler prédéterminé ($D_{th}$), chacune des deux composantes de fréquences ($\omega_1$, $\omega_2$) avec un seuil d'erreur prédéterminé (Cth),

   comparer (S508) quand au moins une des deux composantes de fréquences ($\omega_1$, $\omega_2$) est plus grande que le seuil d'erreur prédéterminé ($C_{th}$), chacune des deux composantes de fréquences ($\omega_1$, $\omega_2$), avec la composante de fréquence moyenne ($\omega_3$), pour détecter (S510) les composantes de fréquence à proximité de la composante de fréquence moyenne ($\omega_3$), comme les composantes de fréquences correspondant aux signaux parasites,

   fixer (S506), quand les deux composantes de fréquences ($\omega_1$, $\omega_2$) sont inférieures au seuil d'erreur prédéterminé ($C_{th}$), la composante de fréquence moyenne ($\omega_3$), comme composante de fréquence correspondant au signal parasite, et,

   effectuer le filtrage des données ultrasoniques en utilisant les composantes de fréquence correspondant au signal parasite.

2. Système ultrasonique (100) selon la revendication 1, dans lequel l'unité de traitement (130) est configurée pour:

   fixer les composantes de fréquence correspondant au signal d'erreur comme composante de fréquence pour abaisser les fréquences,

   effectuer un abaissement des fréquences des données ultrasoniques sur la base de la fréquence d'abaissement des fréquences, et

   effectuer le filtrage des parasites sur la base de la fréquence d'abaissement de la fréquence des signaux des données ultrasoniques,

3. Procédé pour effectuer le filtrage des parasites dans un système ultrasonique (100), agencé pour:

   effectuer une opération de transmission/réception comprenant la transmission de signaux ultrasoniques sur un objet cible la réception des échos ultrasoniques réfléchis par l'objet cible afin d'acquérir des données ultrasoniques pour obtenir une image Doppler en couleur, et

   extraire (S406) deux composantes de fréquences ($\omega_1$, $\omega_2$) provenant des données ultrasoniques utilisant un modèle autoregressif de second ordre, dans lequel les composantes de fréquences ($\omega_1$, $\omega_2$) correspondent aux deux pôles de la fonction modèle du modèle autoregressif,

   **caractérisé en ce qu'**il est en outre configuré pour:

   calculer (S408) une composante de fréquence moyenne. ($\omega_3$) des données ultrasoniques utilisant l'autocorrélation ou la transformation de Fourrier rapide, comparer (S502) chacune des composantes de fréquences ($\omega_1$, $\omega_2$) avec un seuil Doppler prédéterminé ($D_{th}$);

   déterminer quand les deux composantes de fréquences ($\omega_1$, $\omega_2$) sont supérieures au seuil Doppler prédéterminé ($D_{th}$), et qu'un signal parasite n'existe pas dans les données ultrasoniques;

comparer (S504) quand au moins une des deux composantes de fréquences ($\omega_1$, $\omega_2$) est inférieure au seuil Doppler prédéterminé ($D_{th}$), chacune des deux composantes de fréquences ($\omega_1$, $\omega_2$) avec un seuil d'erreur prédéterminé ($C_{th}$);

comparer (S508) quand au moins une des deux composantes de fréquences ($\omega_1$, $\omega_2$) est plus grande que le seuil d'erreur prédéterminé ($C_{th}$), chacune des deux composantes de fréquences ($\omega_1$, $\omega_2$), avec la composante de fréquence moyenne ($\omega_3$), pour détecter (S510) les composantes de fréquence à proximité de la composante de fréquence moyenne ($\omega_3$), comme les composantes de fréquences correspondant aux signaux parasites;

fixer (S506), quand les deux composantes de fréquences ($\omega_1$, $\omega_2$) sont inférieures au seuil d'erreur prédéterminé ($C_{th}$), la composante de fréquence moyenne ($\omega_3$), comme composante de fréquence correspondant au signal parasite, et,

effectuer le filtrage des données ultrasoniques en utilisant les composantes de fréquence correspondant au signal parasite.

4. Procédé selon la revendication 3, comportant en outre:

la fixation des composantes de fréquence correspondant au signal d'erreur comme composante de fréquence pour abaisser les fréquences,

l'abaissement des fréquences des données ultrasoniques sur la base de la fréquence d'abaissement des fréquences, et

le filtrage des parasites sur la base de la fréquence d'abaissement de la fréquence des signaux des données ultrasoniques.

# FIG. 1

110

100

USER INPUT
UNIT

120

130    150

ULTRASOUND
DATA ACQUISITION
UNIT

PROCESSING
UNIT

DISPLAY
UNIT

STORAGE
UNIT

140

# FIG. 2

# FIG. 3

# FIG. 4

```
        ┌─────────┐
        │  START  │
        └─────────┘
             │
             ▼
┌──────────────────────────┐
│   FORM B-MODE IMAGE      │──── S402
└──────────────────────────┘
             │
             ▼
┌──────────────────────────┐
│        SET ROI           │──── S404
└──────────────────────────┘
        │            │
S406    ▼            ▼    S408
┌──────────────────┐  ┌──────────────────────┐
│ APPLY AUTOREGRESSIVE │  │   AUTOCORRELATION   │
│      MODEL         │  └──────────────────────┘
└──────────────────┘
        │            │
        └─────┬──────┘
              ▼
┌──────────────────────────────┐
│    DETECT FREQUENCY          │
│ COMPONENTS CORRESPONDING     │──── S410
│    TO CLUTTER SIGNAL         │
└──────────────────────────────┘
              │
              ▼
┌──────────────────────────────┐
│    PERFORM FILTERING         │──── S412
└──────────────────────────────┘
              │
              ▼
┌──────────────────────────────┐
│  FORM COLOR DOPPLER IMAGE    │──── S414
└──────────────────────────────┘
              │
              ▼
        ┌─────────┐
        │   END   │
        └─────────┘
```

# FIG. 5

```
                    ┌─────────┐
                    │  START  │
                    └─────────┘
                         │
                         ▼           S502
         YES    ╱◇─────────────◇╲
    ┌─────────── ◇  W₁, W₂ > D th ? ◇
    │           ╲◇─────────────◇╱
    │                 │ NO    S504
    │                 ▼
    │         ╱◇─────────────◇╲      YES                        S506
    │         ◇  W₁, W₂ < C th ? ◇──────────────┐
    │         ╲◇─────────────◇╱                 ▼
    │                 │ NO          ┌──────────────────────────────┐
    │                 │             │ DETERMINE W₃ AS FREQUENCY     │
    │                 │             │ COMPONENTS CORRESPONDING      │
    │                 │             │ TO CLUTTER SIGNAL             │
    │                 │             └──────────────────────────────┘
    │                 ▼
    │        ┌──────────────────────────┐
    │        │ COMPARE W₁, W₂ WITH W₃    │─── S508
    │        └──────────────────────────┘
    │                 ▼
    │        ┌──────────────────────────┐
    │        │ DETERMINE PROXIMATE       │
    │        │ FREQUENCY COMPONENTS      │─── S510
    │        │ TO W₃ AS FREQUENCY        │
    │        │ COMPONENTS CORRESPONDING  │
    │        │ TO CLUTTER SIGNAL         │
    │        └──────────────────────────┘
    │                 ▼
    │            ┌─────────┐
    └──────────► │   END   │
                 └─────────┘
```

Decision S502: $W_1, W_2 > D_{th}$ ?  — YES / NO

Decision S504: $W_1, W_2 < C_{th}$ ?  — YES / NO

S506: DETERMINE $W_3$ AS FREQUENCY COMPONENTS CORRESPONDING TO CLUTTER SIGNAL

S508: COMPARE $W_1, W_2$ WITH $W_3$

S510: DETERMINE PROXIMATE FREQUENCY COMPONENTS TO $W_3$ AS FREQUENCY COMPONENTS CORRESPONDING TO CLUTTER SIGNAL

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **Y. B. AHN.** *Estimation of Mean Frequency and Variance of Ultrasonic Doppler Signal by Using Second-Order Autoregressive Model*
- **X. WANG.** *A Novel Clutter Rejection Filters Applied to Wideband Blood Flow Velocity Estimation*

- **P. WANG ; XIAOTAO WANG et al.** *An Improved Mean Frequency Estimator for Ultrasonic Color Flow Imaging Using Second-Order Autoregressive Model*